# EUROPEAN PATENT SPECIFICATION

(11) **EP 3 548 001 B1**
(45) Date of publication and mention of the grant of the patent: **13.04.2022**
(21) Application number: 16816395.4
(22) Date of filing: 30.11.2016
(51) Int. Cl.: A61K 9/08, A61K 47/42, A61K 9/51, A61K 31/47, A61K 31/616, A61K 31/7048

(54) **A METHOD OF MANUFACTURING A SOLUTION OF NANOPARTICLES OF ACETYLSALICYLIC ACID OR DIOSMIN OR DROTAVERINE**
METHODE ZUR HERSTELLUNG EINER LÖSUNG VON NANOPARTIKELN VON ACETYLSALICYLSÄURE ODER DIOSMIN ODER DROTAVERIN
PROCÉDÉ DE FABRICATION D'UNE SOLUTION DE NANOPARTICULES D'ACIDE ACÉTYLSALICYLIQUE, DE DIOSMINE OU DE DROTAVÉRIN

(43) Date of publication of application: 09.10.2019
(73) Proprietor: Dukebox SP. Z O.O., 01-059 Warszawa (PL)
(72) Inventor: BANACH, Marcin, 32-100 Proszowice (PL); PULIT-PROCIAK, Jolanta, 31-866 Krakow (PL)
(74) Representative: Krekora, Magdalena
(86) International application number: PCT/IB2016/057230
(87) International publication number: WO 2018/100413

(56) References cited:
- EP-A1- 2 540 287
- EP-A1- 2 578 209
- EP-A2- 1 944 019
- WO-A1-2012/059936
- US-A- 5 118 528

## Description

### TECHNICAL FIELD

The object of the present invention is a method of manufacturing a solution of nanoparticles of acetylsalicylic acid or diosmin or drotaverine.

### BACKGROUND ART

Acetylsalicylic acid is a nonsteroidal anti-inflammatory drug used to treat pain, fever, and inflammation. Given shortly after a heart attack it decreases the risk of death. Acetylsalicylic acid is also used long-term to help prevent heart attacks, strokes, and blood clots in people at high risk.

Diosmin is a flavone, a member of the flavonoid family. Diosmin is used for treating various disorders of blood vessels including hemorrhoids, varicose veins, poor circulation in the legs (venous stasis), and bleeding in the eye or gums. It is also used to treat swelling of the arms (lymphedema) following breast cancer surgery, and to protect against liver toxicity.

Drotaverine ((Z)-1-(3,4-diethoxybenzylidene)-6,7-diethoxy-1,2,3,4-tetrahydroisoquinoline) is an antispasmodic drug prescribed for pain and dysfunction caused by smooth muscle spasm.

Patent application US20080075667 discloses methods for synthesizing the nanoparticles, composite nanoparticles, and hollow nanocapsules. For example, in an exemplary aspect, the nanoparticles (in the form of solid nanogels) may be formed with an optional active ingredient using inverse emulsion polymerization. The nanoparticles may be sonicated in order to control the particle size. The composite nanoparticles may be formed by polymerizing a monomer on the nanoparticle template to form a composite nanoparticle comprising the shell and the nanoparticle template. The hollow nanoparticles may then be formed by partially or fully removing the template, for example using a suitable etchant. Active ingredients which can be used by the claimed methods include aspirin, acetaminophen, ibuprofen, naproxen sodium and the like.

Patent application WO2012059936 discloses pharmaceutical compositions - an aqueous liquid composition and a non-aqueous liquid composition which on mixing form drug loaded heterogeneous nanosystems of average size <1µm. The aqueous liquid composition comprises polymer/s biodegradable and non-biodegradable, surfactants include cationic, anionic and nonionic, surface modifying agents including hydrophilic polymers, carbohydrate, proteins and receptor specific ligands, pH modulators includes buffers, acid and bases, stabilizers include antioxidants, absorption enhancers, targeting ligands. The non-aqueous liquid composition comprises surfactant/s cationic, anionic and nonionic, polymer/s biodegradable and non-biodegradable, lipid s fatty acids and glycerol esters of fatty acids and at least one non-aqueous solvent. Active agent is dissolved in either aqueous composition or non-aqueous composition. The drug loaded heterogeneous nanosystems of said inventions are generated by mixing the aqueous and non-aqueous compositions in a predetermined ratio. The aqueous phase may be selected from water, water comprising of one or more buffering agents, sodium salts such as sodium chloride, sodium phosphate, sodium dihydrogen phosphate, disodium hydrogen phosphate, potassium salts such as potassium phosphate, potassium dihydrogen phosphate, dipotassium hydrogen phosphate sugars and sugar alcohols such as mannitol, sorbitol, dextrose. Optionally the aqueous phase may be adjusted to isotonic or hypertonic. The composition may comprise diosmin, drotaverine or acetylsalicylic acid (aspirin).

May S. Freag, Yosra S.R. Elnaggar, Ossama Y. Abdallah Development of novel polymer-stabilized diosmin nanosuspensions: In vitro appraisal and ex vivo permeation, International Journal of Pharmaceutics 454(1) July 2013 describes diosmin nanosuspension prepared with acid base neutralization technique. DSN (250 mg) was dissolved in 1 N NaOH (5 mL) then the alka-line solution of DSN (5%, w/v) was added drop wise onto 50 mL0.1 N HCI containing polymeric stabilizer (hydroxypropylmethyl-cellulose HPMC or methylcellulose MC) under continuous stirringat 100 rpm. Stirring process was continued for 30 min after addition till homogenous milky suspension (0.456%, w/v) was obtained.

### DISCLOSURE OF THE INVENTION

The method in accordance with the present invention solves the problem of preparation of solutions of acetylsalicylic acid or diosmin or drotaverine and thus improving their bioavailability and efficacy.

A method of manufacturing a solution of nanoparticles of acetylsalicylic acid or diosmin or drotaverine of the present invention consists in that, acetylsalicylic acid or diosmin or drotaverine is dissolved in an organic solvent, and at least one dispersing agents is dissolved in water, then the organic solution is added to the aqueous solution and the obtained mixture is homogenised through the use of ultrasonic frequencies until homogenous solution is obtained. The method leads to obtaining dispersed nanoparticles of the active substance, whose average diameter is 50 to 500 nm. Obtained solution is a final product, that may be administered to patients as a medicinal product, or may be used as a starting material for manufacture of medicinal products. The solution possesses enhanced bioavailability and is very stable as the nanoparticles do not aggregate. The method is technologically simple, and safe.

Preferably acetylsalicylic acid or diosmin is dissolved in ethanol.

Preferably the solution of acetylsalicylic acid or diosmin in ethanol has concentration of from 70 to 1500 g/dm³.

Preferably diosmin is dissolved in dimethyl sulfoxide (DMSO).

Preferably a solution of diosmin in dimethyl sulfoxide (DMSO) has concentration of from 70 to 1500 g/dm³.

Preferably the concentration of organic solvent in the obtained mixture is not higher than 40%.

Preferably dissolution of dispersing agents in water is carried out in a temperature non higher than 90°C.

Preferably dispersing agents are chosen from a group consisting of lecithin, gelatine and starch.

Preferably the aqueous solution of dispersing agents contains lecithin in concentration from 30 to 135 g/dm³ and gelatin in concentration from 3 to 30 g/dm³.

Preferably the aqueous solution of dispersing agents contains lecithin in concentration from 23 to 135 g/dm³ and starch in concentration from 6 to 135 g/dm³.

Preferably the aqueous solution of dispersing agents contains lecithin in concentration from 19 to 47 g/dm³ and gelatin in concentration from 7 to 16 g/dm³ and starch in concentration from 7 to 16 g/dm³.

Preferably the aqueous solution of dispersing agents contains potassium sorbate.

Preferably the concentration of potassium sorbate is from 3 to 8 g/dm³.

Preferably the strength of ultrasound is from 500 to 600 W.

Preferably the homogenization process is carried out from 1 to 15 minutes.

Preferably the ultrasonication is carried out in flow system or in batch system.

The present invention refers also to a solution of acetylsalicylic acid or diosmin or drotaverine nanoparticles obtained by method according to the invention.

While the organic solution of acetylsalicylic acid or diosmin or drotaverine is introduced into aqueous solution of other ingredients, it is precipitated. Sonication process enables obtaining of particles having nanometric size. This size is preserved as a result of stabilisation activity of lecithin, gelatine and starch. Sonication may be carried out in a batch reactor or in a flow system, each time supplied with a sonication kit having appropriate power - preferably from 500 to 600 W.

### BEST MODE OF CARRYING OUT THE INVENTION

The following examples illustrate the invention without setting or delineating its limits.

### Example 1

Ethanol solution of acetylsalicylic acid was prepared by adding 12.5 g of the acid to 50 cm³ of ethanol (99,6%) and aqueous solution of lecithin, starch and potassium sorbate was prepared by adding 200 cm³ of water to 7 g of lecithin, 2 g of starch and 0.8 g of potassium sorbate. Starch and lecithin were dissolved in the temperature of about 50°C. Then the solution of acetylsalicylic acid was added to the solution of lecithin, starch and potassium sorbate. The volume ratio of both solution was equal to 0.25. Obtained solution underwent sonication. The process of sonication was carried out for 5 minutes at a power of 600W.

The Malvern Zetasizer apparatus using method of dynamic light scattering (DLS) was utilized to measure the average size of particles. Potential zeta was also measured. The solution of acetylsalicylic acid nanoparticles obtained had concentration of about 50 mg/cm³ and the average size of nanoparticles was 109 nm. Potential zeta was equal to -16 mV. The absolute value of the electrokinetic (zeta) potential confirms high stability of the solution.

### Example 2

A solution of diosmin in DMSO was prepared by adding 18 g of diosmin to 90 cm³ of DMSO and aqueous solution of gelatine, lecithin, starch and potassium sorbate was prepared by adding 7 g of lecithin, 2 g of gelatine, 2 g of starch to 300 cm³ of water. Starch, gelatine and lecithin were dissolved in the temperature of about 70°C. Then 0,8 g of potassium sorbate was added to the solution, and the whole mixture was mixed. Afterwards the solution of diosmin was added to the solution of gelatine, starch, lecithin, and potassium sorbate. Obtained solution was treated with ultrasounds for 10 minutes at a power of 500W.

The Malvern Zetasizer apparatus using method of dynamic light scattering (DLS) was utilized to measure the average size of particles. Potential zeta was also measured. The average size of nanoparticles was 220 nm. Potential zeta was equal to -31,4 mV. The absolute value of the electrokinetic (zeta) potential confirms high stability of the solution.

### Example 3

Ethanol solution of drotaverine was prepared by adding 35 g of drotaverine to 50 cm³ ethanol (99,6%) and aqueous solution of lecithin, starch and potassium sorbate was prepared by adding 10 g of lecithin, 2 g of starch and 0.8 g of potassium sorbate to 300 cm³ of water. Lecithin and starch were dissolved in the temperature of about 70°C. Then the solution of drotaverine was added to the solution of lecithin, starch and potassium sorbate. The volume ratio of both solution was equal to 0.17. Obtained solution underwent sonication. The process of sonication was carried out for 10 minutes at a power of 600W.

The Malvern Zetasizer apparatus using method of dynamic light scattering (DLS) was utilized to measure the average size of particles. Potential zeta was also measured. The solution of drotaverine nanoparticles had concentration of about 100 mg/cm³ and the average size of nanoparticles was 281 nm. Potential zeta was equal to -27 mV. The absolute value of the electrokinetic (zeta) potential confirms high stability of the solution.

### Example 4

Ethanol solution of acetylsalicylic acid was prepared by adding 1,76 g of the acid to 50 cm³ ethanol (99,6%) and aqueous solution of gelatine and lecithin was prepared by adding 4 g of lecithin and 0,5 g of gelatine to 125 cm³ of water. Gelatine and lecithin were dissolved in the temperature of about 75°C. Afterwards the solution of acetylsalicylic acid was added to the solution of gelatine and lecithin. The volume ratio of both solution was equal to 0.4. Obtained solution was treated with ultrasounds for 5 minutes at a power of 600W.

The Malvern Zetasizer apparatus using method of dynamic light scattering (DLS) was utilized to measure the average size of particles. Potential zeta was also measured. The solution of acetylsalicylic acid nanoparticles had concentration of about 100 mg/cm³ and the average size of nanoparticles was 220 nm. The size of particles was then measured after 24 hours, and 48 hours. It was equal to 215 nm, and 204 nm. Lack of significant changes of the average nanoparticles size confirms high stability of the solution.

## Claims

1. A method of manufacturing a solution of nanoparticles of acetylsalicylic acid or diosmin or drotaverine, **characterised in that**, acetylsalicylic acid or diosmin or drotaverine is dissolved in an organic solvent, and at least one dispersing agent chosen from a group consisting of lecithin, gelatin and starch is dissolved in water, and then potassium sorbate is added, then the organic solution is added to the aqueous solution and the obtained mixture is homogenised through the use of ultrasonic frequencies until homogenous solution is obtained.

2. A method according to claim 1, **characterized in that**, acetylsalicylic acid or diosmin is dissolved in ethanol.

3. A method according to claim 2, **characterized in that**, solution of acetylsalicylic acid or diosmin in ethanol has concentration of from 70 to 1500 g/dm³.

4. A method according to claim 1, **characterized in that**, diosmin is dissolved in dimethyl sulfoxide (DMSO).

5. A method according to claim 4, **characterized in that**, a solution of diosmin in dimethyl sulfoxide (DMSO) has concentration of from 70 to 1500 g/dm³.

6. A method according to any of the preceding claims, **characterized in that**, the concentration of organic solvent in the obtained mixture is not higher than 40%.

7. A method according to any of the preceding claims, **characterized in that**, dissolution of dispersing agents in water is carried out in a temperature non higher than 90°C.

8. A method according to any of the preceding claims, **characterized in that**, the aqueous solution of dispersing agents contains lecithin in concentration from 30 to 135 g/dm³ and gelatin in concentration from 3 to 30 g/dm³.

9. A method according to any of the claims from 1 to 7, **characterized in that**, the aqueous solution of dispersing agents contains lecithin in concentration from 23 to 135 g/dm³ and starch in concentration from 6 to 135 g/dm³.

10. A method according to any of the claims from 1 to 7, **characterized in that**, the aqueous solution of dispersing agents contains lecithin in concentration from 19 to 47 g/dm³ and gelatin in concentration from 7 to 16 g/dm³ and starch in concentration from 7 to 16 g/dm³.

11. A method according to any of the preceding claims, **characterized in that**, the concentration of potassium sorbate is from 3 to 8 g/dm³.

12. A method according to any of the preceding claims, **characterized in that**, the strength of ultrasound is from 500 to 600 W.

13. A method according to any of the preceding claims, **characterized in that**, the homogenization process is carried out from 1 to 15 minutes.

14. A method according to any of the preceding claims, **characterized in that**, the ultrasonication is carried out in flow system or in batch system.

## Patentansprüche

1. Methode zur Herstellung einer Lösung von Nanopartikeln von Acetylsalicylsäure oder Diosmin oder Drotaverin, **dadurch gekennzeichnet, dass** Acetylsalicylsäure oder Diosmin oder Drotaverin in einem organischen Lösungsmittel gelöst wird und dass zumindest ein Dispergiermittel, das ausgewählt wird aus der Gruppe, bestehend aus Lecithin, Gelatine und Stärke, in Wasser gelöst wird und dass dann Kaliumsorbat hinzugefügt wird, dass dann die organische Lösung zu der wässrigen Lösung hinzugefügt wird und dass das erhaltene Gemisch durch die Verwendung von Ultraschallfrequenzen homogenisiert wird, bis eine homogene Lösung erhalten wird.

2. Methode nach Anspruch 1, **dadurch gekennzeichnet, dass** Acetylsalicylsäure oder Diosmin in Ethanol gelöst wird.

3. Methode nach Anspruch 2, **dadurch gekennzeichnet, dass** die Lösung von Acetylsalicylsäure oder Diosmin in Ethanol eine Konzentration im Bereich von 70 bis 1500 g/dm³ aufweist.

4. Methode nach Anspruch 1, **dadurch gekennzeichnet, dass** Diosmin in Dimethylsulfoxid (DMSO) gelöst wird.

5. Methode nach Anspruch 4, **dadurch gekennzeichnet, dass** eine Lösung von Diosmin in Dimethylsulfoxid (DMSO) eine Konzentration im Bereich von 70 bis 1500 g/dm³ aufweist.

6. Methode nach einem der vorhergehenden Ansprüche, **dadurch gekennzeichnet, dass** die Konzentration von organischem Lösungsmittel im erhaltenen Gemisch nicht höher als 40 % ist.

7. Methode nach einem der vorhergehenden Ansprüche, **dadurch gekennzeichnet, dass** die Auflösung von Dispergiermitteln in Wasser bei einer Temperatur nicht höher als 90 °C ausgeführt wird.

8. Methode nach einem der vorhergehenden Ansprüche, **dadurch gekennzeichnet, dass** die wässrige Lösung von Dispergiermitteln Lecithin in einer Konzentration von 30 bis 135 g/dm³ und Gelatine in einer Konzentration von 3 bis 30 g/dm³ enthält.

9. Methode nach einem der Ansprüche 1 bis 7, **dadurch gekennzeichnet, dass** die wässrige Lösung von Dispergiermitteln Lecithin in einer Konzentration von 23 bis 135 g/dm³ und Stärke in einer Konzentration von 6 bis 135 g/dm³ enthält.

10. Methode nach einem der Ansprüche 1 bis 7, **dadurch gekennzeichnet, dass** die wässrige Lösung von Dispergiermitteln Lecithin in einer Konzentration von 19 bis 47 g/dm³ und Gelatine in einer Konzentration von 7 bis 16 g/dm³ und Stärke in einer Konzentration von 7 bis 16 g/dm³ enthält.

11. Methode nach einem der vorhergehenden Ansprüche, **dadurch gekennzeichnet, dass** die Konzentration von Kaliumsorbat im Bereich von 3 bis 8 g/dm³ ist.

12. Methode nach einem der vorhergehenden Ansprüche, **dadurch gekennzeichnet, dass** die Stärke des Ultraschalls im Bereich von 500 bis 600 W liegt.

13. Methode nach einem der vorhergehenden Ansprüche, **dadurch gekennzeichnet, dass** der Homogenisierungsprozess zwischen 1 und 15 Minuten lang ausgeführt wird.

14. Methode nach einem der vorhergehenden Ansprüche, **dadurch gekennzeichnet, dass** die Ultraschallbehandlung in einem kontinuierlichen System oder einem diskontinuierlichen System ausgeführt wird.

## Revendications

1. Procédé de fabrication d'une solution de nanoparticules d'acide acétylsalicylique, de diosmine ou de drotavérine, **caractérisé en ce que** l'acide acétylsalicylique, la diosmine ou la drotavérine est dissous dans un solvant organique et au moins un agent dispersant choisi dans le groupe constitué par la lécithine, la gélatine et l'amidon est dissous dans de l'eau, et puis on ajoute du sorbate de potassium, ensuite la solution organique est ajoutée à la solution aqueuse et le mélange obtenu est homogénéisé par utilisation de fréquences ultrasonores jusqu'à obtention d'une solution homogène.

2. Procédé selon la revendication 1, **caractérisé en ce que** l'acide acétylsalicylique ou la diosmine est dissous dans l'éthanol.

3. Procédé selon la revendication 2, **caractérisé en ce que** la solution d'acide acétylsalicylique ou de diosmine dans l'éthanol a une concentration allant de 70 à 1500 g/dm³.

4. Procédé selon la revendication 1, **caractérisé en ce que** la diosmine est dissoute dans le diméthylsulfoxyde (DMSO).

5. Procédé selon la revendication 4, **caractérisé en ce que** la solution de diosmine dans le diméthylsulfoxyde (DMSO) a une concentration allant de 70 à 1500 g/dm³.

6. Procédé selon l'une quelconque des revendications précédentes, **caractérisé en ce que** la concentration de solvant organique dans le mélange obtenu est inférieure ou égale à 40%.

7. Procédé selon l'une quelconque des revendications précédentes, **caractérisé en ce que** la dissolution des agents dispersants dans l'eau s'effectue à une température ne dépassant pas 90°C.

8. Procédé selon l'une quelconque des revendications précédentes, **caractérisé en ce que** la solution aqueuse d'agents dispersants contient de la lécithine à une concentration allant de 30 à 135 g/dm3 et de la gélatine à une concentration allant de 3 à 30 g/dm³.

9. Procédé selon l'une quelconque des revendications 1 à 7, **caractérisé en ce que** la solution aqueuse d'agents dispersants contient de la lécithine à une concentration allant de 23 à 135 g/dm3 et de l'amidon à une concentration allant de 6 à 135 g/dm³.

10. Procédé selon l'une quelconque des revendications 1 à 7, **caractérisé en ce que** la solution aqueuse d'agents dispersants contient de la lécithine à une concentration allant de 19 à 47 g/dm3, de la gélatine à une concentration allant de 7 à 16 g/dm3 et de l'amidon à une concentration allant de 7 à 16 g/dm³.

11. Procédé selon l'une quelconque des revendications précédentes, **caractérisé en ce que** la concentration en sorbate de potassium va de 3 à 8 g/dm³.

12. Procédé selon l'une quelconque des revendications précédentes, **caractérisé en ce que** la puissance des ultrasons va de 500 à 600 W.

13. Procédé selon l'une quelconque des revendications précédentes, **caractérisé en ce que** le processus d'homogénéisation est effectué pendant 1 à 15 minutes.

14. Procédé selon l'une quelconque des revendications précédentes, **caractérisé en ce que** l'ultrasonication s'effectue selon un processus continu ou discontinu.
